# EUROPEAN PATENT SPECIFICATION

(11) **EP 0 900 196 B1**
(45) Date of publication and mention of the grant of the patent: **02.03.2005**
(21) Application number: 97953815.4
(22) Date of filing: 15.12.1997
(51) Int. Cl.: C07C 231/02

(54) **A PROCESS FOR THE PREPARATION OF AN INTERMEDIATE USEFUL IN THE SYNTHESIS OF IODINATED CONTRAST MEDIA**
VERFAHREN ZUR HERSTELLUNG EINES ZWISCHENPRODUKTES FÜR DIE SYNTHESE VON IODIERTEN KONTRASTMITTELN
PROCEDE DESTINE A LA PREPARATION D'UN PRODUIT INTERMEDIAIRE UTILE POUR LA SYNTHESE DE MILIEUX DE CONTRASTE IODES

(30) Priority: 24.12.1996 IT MI962736
(43) Date of publication of application: 10.03.1999
(73) Proprietor: Bracco Imaging S.p.A., 20134 Milano (IT)
(72) Inventor: VILLA, Marco, I-20125 Milano (IT); PAIOCCHI, Maurizio, I-20152 Milano (IT)
(74) Representative: Minoja, Fabrizio, Dr.
(86) International application number: PCT/EP1997/007026
(87) International publication number: WO 1998/028259

(56) References cited:
- WO-A-96/37460
- CH-A- 616 403
- GB-A- 1 472 050
- GB-A- 2 271 990

## Description

The present invention relates to a process for the preparation of an intermediate useful in the synthesis of iodinated contrast media, more particularly, to a process for the preparation of the compound L-5-(2-acetoxypropionylamino)-2,4,6-triiodo-isophthaloyl dichloride (hereinafter referred to as compound A). Compound A, disclosed in British Patent n° 1,472,050 (Savac AG), is an intermediate for the synthesis of (S)-N,N'-bis[2-hydroxy-(1-hydroxymethyl)ethyl]-5-(2-hydroxypropionylamino)-2,4,6-triiodo-isophthalamide, an X-ray non-ionic contrast medium, also known under the international non-proprietary name Iopamidol.

The preparation of compound A as disclosed in the above cited British Patent (example 1b) is performed adding L-2-acetoxypropionyl chloride to a solution of 5-amino-2,4,6-triiodo-isophthaloyl dichloride in N,N-dimethylacetamide at 3-5°C according to the following scheme.

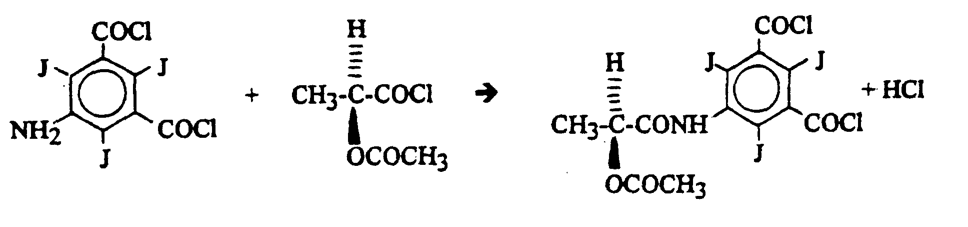

The reaction described in example 1b of British Patent n° 1,472,050 has some drawbacks - in particular a partial racemization of L-2-acetoxypropionyl chloride - which make it industrially unsuitable. In this connection, it should be stressed that through the reaction between 5-amino-2,4,6-triiodo-isophthaloyl dichloride and L-2-acetoxypropionyl chloride the stereogenic center present in the Iopamidol molecule is inserted. The purity requirements of Iopamidol as an X-ray contrast medium involve of course also the optical purity of the compound itself (specific rotatory power from -4.6° to -5.2°), reported in the "Farmacopea Ufficiale della Repubblica Italiana", (Italian Official Pharmacopoeia, Ninth edition, II Supplement, 1991, page 429), corresponding to an e. e. ≥ 89%.

Therefore it is of paramount importance that, starting from the reaction for the preparation of compound A, the enantiomeric purity of the various synthetic intermediates be maintained high in order to avoid the need for a purification of the final product from the undesired enantiomer. To this purpose, the reaction for the preparation of compound A makes use of L-2-acetoxypropionyl chloride with a high enantiomeric purity, generally with an e. e. ≥ 95%, preferably higher than 97%.

Improvements to the process described above were recently reported in literature, which involve the addition of catalytic amounts of acids to the reaction mixture. In particular, the addition of catalytic amounts of Lewis acids has been described in British Patent application n° 2,271,990 in the Applicant's name, while the addition of catalytic amounts of halo acids has been described in International Patent application n. WO 96/37460 in the name of Fructamine S.p.a.

Now it has been found that the same improvements can be attained in the preparation of compound A, mainly as far as the reduction in the racemization of L-2-acetoxypropionyl chloride is concerned, while avoiding the addition of some acids to the reaction mixture.

It is therefore the object of the present invention a process for the preparation of L-5-(2-acetoxypropionylamino)-2,4,6-triibdo-isophthaloyl dichloride by reaction of 5-amino-2,4,6-triiodoisophthaloyl dichloride with L-2-acetoxypropionyl chloride in N,N-dimethylacetamide as a solvent, in which process a catalytic amount of water is added to the reaction mixture.

The improved process of the present invention allows to obtain compound A with a high enantiomeric purity, i.e. with an e. e. higher than 92%. Racemization is thus minimized and the resulting compound A has an enantiomeric purity degree suitable for using it directly as a crude in the synthesis of Iopamidol, thus obtaining Iopamidol with a rotatory power fulfilling the requirements of Pharmacopoeia.

In practice, since the reaction between L-2-acetoxypropionyl chloride and 5-amino-2,4,6-triiodoisophthaloyl dichloride is carried out using an L-2-acetoxypropionyl chloride excess, the catalytic amount of water is meant on the basis of 5-amino-2,4,6-triiodoisophthaloyl dichloride, and specifically it ranges from 5% to 50% per mole of 5-amino-2,4,6-triiodoisophthaloyl dichloride. Preferably, the amount of water ranges from 10% to 30% per mole of 5-amino-2,4,6-triiodoisophthaloyl dichloride.

Water is preferably added to the solution of L-2-acetoxypropionyl chloride in N,N-dimethylacetamide before the addition of the 5-amino-2,4,6-triiodoisophthaloyl dichloride. The reaction temperature is generally low, preferably from 0°C to 25°C. Temperature is not a critical parameter. However, operating at lower temperatures, racemization is further limited but the reaction rate is lower. On the contrary, when operating at too high temperatures, racemization is liable to increase.

Although the process of the invention is illustrated with particular reference to N,N-dimethylacetamide as the solvent, the process can be carried out similarly in other dipolar aprotic solvents such as N-methyl-2-pyrrolidone.

As already noted, the process of the invention minimizes racemization to values which allow to obtain compound A with an enantiomeric purity degree suitable for the preparation of Iopamidol fulfilling the requirements of Pharmacopoeia, without need for further purifications.

A preferred embodiment of the process of the invention is described in the following. A catalytic amount of water is added to a solution of L-2-acetoxypropionyl chloride in N,N-dimethylacetamide, then 5-amino-2,4,6-triiodo-isophthaloyl dichloride is added thereto. After that, the mixture is kept at room temperature for about 12-16 hours. The reaction mixture is treated with cellosolve and water to obtain a precipitate which is filtered, washed and dried to obtain compound A with an enantiomeric purity substantially corresponding to that of the starting L-2-acetoxypropionyl chloride.

A further object of the invention is a process for the preparation of Iopamidol, characterized in that the intermediate L-5-(2-acetoxypropionylamino)-2,4,6-triiodoisophthaloyl dichloride is obtained by reacting 5-amino-2,4,6-triiodoisophthaloyl dichloride with L-2-acetoxypropionyl chloride in the presence of N,N-dimethylacetamide as the solvent, adding a catalytic amount of water to the reaction mixture.

The present invention is further illustrated by the following examples.

### Example 1

A 250 ml round-bottomed flask, fitted with mechanical stirring and kept under inert atmosphere, is loaded with N,N-dimethylacetamide (30 ml), then cooled at -2 - 0°C and L-2-acetoxypropionyl chloride (20 g; 0.133 moles; e. e. 97.4%) is added drop by drop in an hour. When the addition is completed, water (0.360 g; 20.1 mmoles) is added. Temperature is adjusted to 12°C and a solution of N,N-dimethylacetamide (30 ml) and 5-amino-2,4,6-triiodoisophthaloyl dichloride (40 g; 67 mmoles) is dropped therein in 6 hours. After that, the solution is warmed to 19-20°C, keeping this temperature for 14-16 hours. The mixture is cooled to 5-10°C and cellosolve (40 ml) is added, keeping temperature lower than 20°C. The solution is added drop by drop to water (280 ml) in about 1 hour, keeping temperature at 15-20°C. The suspension is stirred for 30 minutes and the precipitate is filtered, washed with water (2x30 ml) and dried under vacuum at 50°C until constant weight to obtain 42 g of the desired product. Titre ≥ 97%, 95% e. e., 89% yield.

## Claims

1. A process for the preparation of L-5-(2-acetoxypropionylamino)-2,4,6-triiodoisophthaloyl dichloride by reacting 5-amino-2,4,6-triiodoisophthaloyl dichloride with an excess of L-2-acetoxypropionyl chloride in a dipolar aprotic solvent **characterized in that** from 5% to 50% of water per mole of 5-amino-2,4,6-triiodoisophthaloyl dichloride is added to the reaction mixture.

2. A process according to claim 1, wherein the dipolar aprotic solvent is N,N-dimethylacetamide.

3. A process according to claims 1-2 wherein the amount of water ranges from 10% to 30% per mole of 5-amino-2,4,6-triiodoisophthaloyl dichloride.

4. A process according to claims 2-3, wherein water is added to a solution of L-2-acetoxypropionyl chloride in N,N-dimethylacetamide and then 5-amino-2,4,6-triiodo-isophtaloyl dichloride is added thereto.

5. A process according to Claim 2 for the preparation of Iopamidol which comprises the preparation of L-5-(2-acetoxypropionylamino)-2,4,6-triiodoisophthaloyl dichloride by reacting 5-amino-2,4,6-triiodoisophthaloyl dichloride with L-2-acetoxypropionyl chloride in the presence of N,N-dimethylacetamide as the solvent, adding from 5% to 50% of water per mole of 5-amino-2,4,6-triiodoisophthaloyl dichloride to the reaction mixture.

## Patentansprüche

1. Verfahren zur Herstellung von L-5-(2-Acetoxypropionylamino)-2,4,6-triiodisophthaloyldichlorid durch Umsetzen von 5-Amino-2,4,6-triiodisophthaloyldichlorid mit einem überschuss an L-2-Acetoxypropionylchlorid in einem dipolaren, aprotischen Lösungsmittel, **dadurch gekennzeichnet, dass** dem Reaktionsgemisch 5% bis 50% Wasser pro Mol 5-Amino-2,4,6-triiodisophthaloyldichlorid zugegeben werden.

2. Verfahren nach Anspruch 1, wobei das dipolare, aprotische Lösungsmittel N,N-Dimethylacetamid ist.

3. Verfahren nach den Ansprüchen 1 bis 2, wobei die Menge an Wasser von 10% bis 30% pro Mol 5-Amino-2,4,6-triiodisophthaloyldichlorid reicht.

4. Verfahren nach den Ansprüchen 2 bis 3, wobei Wasser zu einer Lösung von L-2-Acetoxypropionylchlorid in N,N-Dimethylacetamid gegeben wird und nachfolgend 5-Amino-2,4,6-triiodisophthaloyldichlorid dazugegeben wird.

5. Verfahren nach Anspruch 2 zur Herstellung von Iopamidol, das die Herstellung von L-5-(2-Acetoxypropionylamino)-2,4,6-triiodisophthaloyldichlorid durch Umsetzen von 5-Amino-2,4,6-triiodisophthaloyldichlorid mit L-2-Acetoxypropionylchlorid in der Gegenwart von N,N-Dimethylacetamid als Lösungsmittel und Zugeben von 5% bis 50% Wasser pro Mol 5-Amino-2,4,6-triiodisophthaloyldichlorid zu dem Reaktionsgemisch umfasst.

## Revendications

1. Procédé pour la préparation de dichlorure de L-5-(2-acétoxypropionylamino)-2,4,6-triiodoisophtaloyle en faisant réagir du dichlorure de 5-amino-2,4,6-triiodoisophtaloyle avec un excès de chlorure de L-2-acétoxypropionyle dans un solvant aprotique dipolaire, **caractérisé en ce que** de 5 % à 50 % d'eau par mole de dichlorure de 5-amino-2,4,6-triiodoisophtaloyle est ajouté au mélange réactionnel.

2. Procédé selon la revendication 1, dans lequel le solvant aprotique dipolaire est le N,N-diméthylacétamide.

3. Procédé selon les revendications 1-2, dans lequel la quantité d'eau va de 10 % à 30 % par mole de dichlorure de 5-amino-2,4,6-triiodoisophtaloyle.

4. Procédé selon les revendications 2-3, dans lequel l'eau est ajoutée à une solution de chlorure de L-2-acétoxypropionyle dans du N,N-diméthylacétamide puis le dichlorure de 5-amino-2,4,6-triiodoisophtaloyle y est ajouté.

5. Procédé selon la revendication 2 pour la préparation d'lopamidol, qui comprend la préparation de dichlorure de L-5-(2-acétoxypropionylamino)-2,4,6-triidoisophtaloyle en faisant réagir du dichlorure de 5-amino-2,4,6-triiodoisophtaloyle avec du chlorure de L-2-acétoxypropionyle en présence de N,N-diméthylacétamide en tant que solvant, en ajoutant de 5 % à 50 % d'eau par mole de dichlorure de 5-amino-2,4,6-triiodoisophtaloyle au mélange réactionnel.
